# EUROPEAN PATENT SPECIFICATION

(11) **EP 0 397 637 B1**
(45) Date of publication and mention of the grant of the patent: **19.06.1996**
(21) Application number: 90870070.1
(22) Date of filing: 14.05.1990
(51) Int. Cl.: C07C 15/40, C07C 5/333, B01J 23/22

(54) **Process for the catalytic dehydrogenation of alkylaromatic hydrocarbons**
Verfahren zur katalytischen Dehydrierung von alkylaromatischen Kohlenwasserstoffen
Procédé de déshydrogénation catalytique d'hydrocarbures alkylaromatiques

(30) Priority: 12.05.1989 BE 8900512
(43) Date of publication of application: 14.11.1990
(73) Proprietor: FINA RESEARCH S.A., B-7181 Seneffe (Feluy) (BE)
(72) Inventor: Delorme, Luc Florent Léon, B-1410 Waterloo (BE); Martins Mendes Cerejo, Francisco, B-7190 Ecaussines (BE); Grootjans, Jacques François, B-3061 Leefdaal (BE)

(56) References cited:
- FR-A- 827 068
- GB-A- 840 082
- US-A- 3 118 007
- US-A- 4 607 129
- US-A- 4 644 089

## Description

The present invention relates to a process for the catalytic dehydrogenation of hydrocarbons. Particularly the present invention relates to the dehydrogenation of alkylaromatic hydrocarbons to produce the corresponding alkenylaromatic hydrocarbons. More particularly the process of the invention uses catalytic systems of the redox type.

Catalytic dehydrogenation of hydrocarbons has been carried out for many years and constitutes an important catalytic process in view of the increasing demand in dehydrogenated products which may be valorized under the most various forms such as high octane gasolines, plastic materials and synthetic rubbers.

In the field of dehydrogenation of alkylaromatic hydrocarbons, known processes include thermal dehydrogenation, catalytic dehydrogenation in the presence of an inert diluent such as steam, and oxidative dehydrogenation, the latter involving the injection of molecular oxygen in the reaction medium. However, although the oxidative dehydrogenation may have same advantages regarding reaction yield and selectivity of the desired product, it is also well known that the presence of molecular oxygen in the reaction medium leads to the formation of indesirable oxidation products such as aldehydes.

In view to partially obviate these drawbacks, it has often been proposed to use very specific catalysts having a particular selectivity towards oxidative dehydrogenation of certain hydrocarbons whether or not of the alkylaromatic type.

In this respect, it has already been proposed in US patent 4,777,319 to Kung et al. to use vanadates or mobybdates of metals for the selective dehydrogenation of paraffinic hydrocarbons having from 3 to 6 carbon atoms. However, the dehydrogenation reaction is necessarily carried out in the presence of molecular oxygen, which displaces the thermodynamic equilibrium but leads to the formation of secondary oxidation products.

It has also been proposed in US 4,742,180 to Gaffney to use supported catalysts, the support being essentially an oxide of praseodymium on which there is deposited an alkaline metal having a dehydrogenating action. However, the availability of praseodymium oxide for the production of huge amounts of alkenylaromatic hydrocarbons has to be taken into account. Moreover, it has to be noted that no significant result is indicated for the dehydrogenation of alkylaromatic hydrocarbons. Oganowski has also proposed to use a catalyst of the Mg₃(VO₄)₂ type for the dehydrogenation of ethylbenzene, but the reaction necessarily occurs in the presence of a gas containing molecular oxygen. Moreover, it is known that oxides of the V₂O₅ type lead to reaction of complete combustion when used in dehydrogenation reaction of hydrocarbons carried out without bringing in molecular oxygen.

US-A-3118007 discloses a process for the dehydrogenation of gaseous hydrocarbons using iron oxide containing catalysts in a fluidised bed.

US-A-4644089 discloses a process for the dehydrogenation of C₂₋₂₀ alkanes and C₅₋₂₀ cycloalkanes with a catalyst composition comprising at least one oxide of vanadium and aluminium phosphate.

FR-A-827068 discloses a process for the dehydrogenation of aliphatic hydrocarbons to form mono- or di-olefins.

GB-A-840082 discloses a process for the dehydrogenation of aliphatic mono-olefins into the corresponding di-olefins.

An object of the present invention is to provide an improved process for the catalytic dehydrogenation of alkylaromatic hydrocarbons in the presence of a redox catalytic system.

Another object of the present invention is to provide an improved process for the catalytic dehydrogenation of alkylaromatic hydrocarbons in the absence of molecular oxygen.

Still a further object of the present invention is to provide a continuous process for the dehydrogenation of alkylaromatic hydrocarbons in the presence of a redox catalytic system.

The present invention provides for a process for the catalytic dehydrogenation of alkylaromatic hydrocarbons into corresponding alkenylaromatic hydrocarbons which comprises :
- contacting the hydrocarbon feedstock to be dehydrogenated under dehydrogenation conditions, in the absence of added water vapour and of any gas containing molecular oxygen, with a catalyst consisting of at least one reducible oxide of a metal (I) selected from the group consisting of V, Cr, Mn, Co, Pb, Bi, Mo, U and Sn, supported on a material selected from clays, zeolitic materials of the metallo-silicate or metallo-alumino-phosphate type, and oxides of a metal (II) selected from Ti, Zr, Zn, Th, Mg, Ca, Ba, Si and Al, said catalyst having a dehydrogenation activity when said metal (I) has a valency such that it is not in its most reduced state,
- recovering the dehydrogenated hydrocarbons,
- regenerating the used catalyst, and
- contacting the regenerated catalyst with fresh hydrocarbon feedstock to be dehydrogenated.

The Applicant has now found that with the process of the invention, the presence of the molecular oxygen to perform the dehydrogenation reaction was no longer necessary, this fact constituting an important advantage over the prior processes.

According to the process of the invention, the feedstock of alkylaromatic hydrocarbons to be dehydrogenated is contacted with a catalyst which has to fulfil several conditions. The catalyst comprises at least a reducible oxide of a metal selected from V, Cr, Mn, Co, Bi, Mo, U and Sn; reducible oxides as used herein mean the hereabove metal oxides which are reduced by contact with hydrocarbons, when operating under dehydrogenation conditions. Moreover, the metal oxide used has to have a dehydrogenating action under the reaction conditions.

Further, the Applicant has found that it is advantageous that these oxides be deposited on a support. By way of examples of suitable supports, it may be cited the oxides of metals selected from Ti, Zr, Zn, Mg, Th, Si, Ca, Ba and Al, as well as clays and zeolitic materials of the metallo-silicate or metallo-alumino-phosphate type. Within the latter type, it may be cited the alumino-silicates, the borosilicates, silico-alumino-phosphates and other analogs.

The supported catalysts used in the process of the invention may also comprise promoters such as alkali or alkaline-earth metals.

The supported catalyst may be prepared according to usual methods such as absorption, precipitation or still impregnation.

Among the various catalysts which may be used in the process of the invention, the Applicant has found that favourable results are obtained with catalysts of the vanadium oxide type deposited on a support comprising magnesium oxide.

The contact between the feedstock to be dehydrogenated and the catalyst may be performed according to different ways; for instance the catalyst particles may be used in a fixed bed, or they may be used in a fluidized bed reactor wherein said particles are circulated and thereafter recovered, what implies a suitable distribution of the particles sizes.

According to an embodiment of the process of the present invention the catalyst is placed in a fixed bed and contacted with the feedstock of alkylaromatic hydrocarbons to be dehydrogenated, in the absence of a molecular oxygen containing gas, at a temperature comprised between 300 and 800°C, in order to maintain the feedstock in the vapor phase. When the activity of the catalyst is reduced over the limits which are currently acceptable, generally a reduction of about 10% of the conversion, the feedstock is no longer contacted with the catalyst, but an air stream is passed over the catalyst bed at a temperature of from 200°C to 1000°C in order to regenerate said catalyst under mild conditions. This regeneration comprises at least a mild oxidation of the catalyst. When the catalyst is regenerated the feedstock is again contacted with the catalyst.

According to a preferred embodiment of the present invention, the catalyst particles, whose sizes are comprised between about 0.02 and 0.3 mm, the catalyst being taken in its oxidized form, are circulating in the dehydrogenation reaction zone, and contacted with the feedstock to be dehydrogenated, in the absence of an oxygen containing gas, at a temperature comprised between 300 and 800°C in order to maintain the feedstock in the vapor phase. The gas pressure at the outlet of the reactor is generally comprised between 10⁵ and 1.3 10⁵ Pa while residence time of the feedstock in the reactor is of 0.5 to 15 seconds, while the residence time of the catalyst is comprised between 0.5 second and 5 minutes; the upper limit of the residence time of the catalyst depends obviously on its activity. According to this preferred embodiment, the dehydrogenation reaction and the transportation of the catalyst to the regenerator are more particularly carried out in a fluidized bed reactor.

The catalyst in the reactor effluent is separated from the hydrocarbon effluent by suitable means. The separated catalyst is a reduced catalyst because it is in a lower oxidation state than that of the fresh catalyst which enters the reaction zone. The separated catalyst is sent to a regeneration zone where it is regenerated with gaseous stream containing molecular oxygen. This regeneration comprises at least a mild oxidation of the catalyst. The temperature in the regeneration zone is most often maintained between about 200 and about 1000°C; the residence time of the catalyst in said zone is of about 5 seconds to 5 minutes, while that of the gas containing molecular oxygen is of about 1 to 30 seconds. The amount of gas together with the oxygen concentration must be sufficient to reoxidize the catalyst in its initial form. If this embodiment is used for the regeneration of the catalyst, then the dehydrogenation process may be carried out continuously, while it was not the case with the previous embodiment.

Among the various catalysts that may be used, the Applicant has found that favourable results are obtained, more particularly in the fluidised bed embodiment, with catalysts of the MgO - SiO₂ - V₂O₅ type which may also comprise promoters such as alkali or alkaline-earth metals. These catalysts comprise 40-60 wt % (preferably about 50 %) MgO, 20-40 wt % (preferably about 30 %) SiO₂, 10-30 wt % (preferably about 15 %) V₂O₅, and minor amounts of promoters (typically about 0.5 wt % CaO and about 1.5 wt % Na₂O) and of other impurities. They are preferably prepared by spray-drying of an MgO/SiO₂ suspension, followed by impregnation with an ammonium vanadate solution preferably also with a further amount of SiO₂ as silicasol, evaporation and calcination at a temperature of from about 500°C to about 800°C.

The process of the invention is suitable for the dehydrogenation of alkylaromatic hydrocarbons into corresponding alkenylaromatic hydrocarbons. Particularly, the process of the invention is applied to the catalytic dehydrogenation of ethylbenzene and ethyltoluene into styrene or vinyltoluene respectively. The process is generally carried out at a temperature comprised between 350 and 800°C and preferably between 400 and 650°C, at a pressure comprised between 0.001 and 1 MPa, and at a hourly space velocity comprised between about 0.01 and 20.0 kg of hydrocarbon per hour and per kg of catalyst, preferably between 1 and 10.

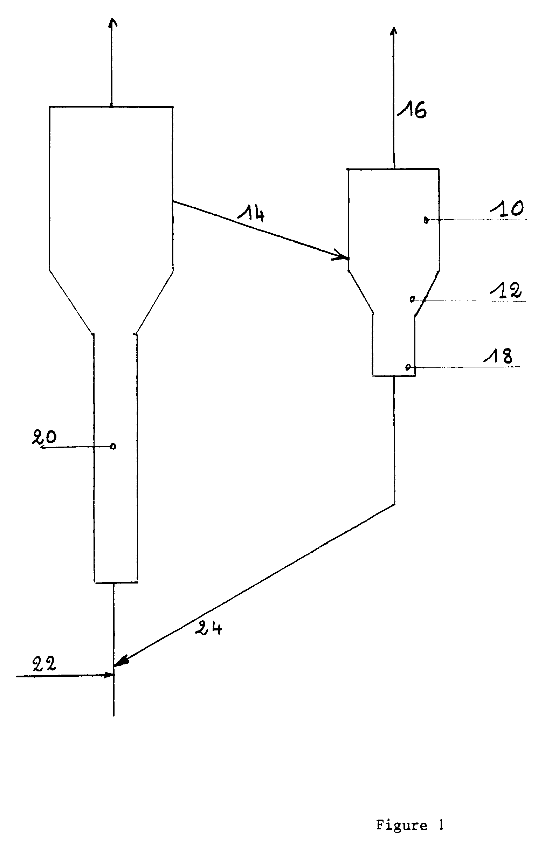

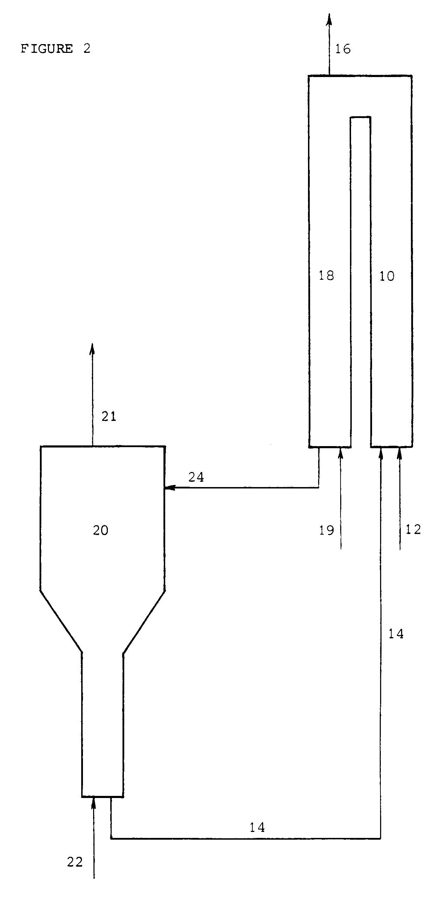

Preferred embodiments of the invention are also described by way of the drawings according to which Figures 1 and 2 represent schematic diagrams of the reaction zone and the regeneration zone of the catalyst.

Referring now to Figure 1, which shows the dehydrogenation reaction zone 10 wherein the feedstock of hydrocarbons to be dehydrogenated enters through pipe 12 while the catalyst under its oxidized form enters through pipe 14. The dehydrogenated hydrocarbons are withdrawn through pipe 16 while the reduced catalyst is collected in the area 18 of the dehydrogenation reactor and transported to the top of the regeneration reactor 20. The gas containing molecular oxygen is introduced into pipe 22 while the reduced catalyst is introduced into pipe 24. The catalyst is recovered in the outlet pipe 14 and transported under its oxidized form in the dehydrogenation reactor 10.

Referring to Figure 2, the feedstock of hydrocarbons to be dehydrogenated enters the dehydrogenation zone 10 through pipe 12 while the catalyst under its oxidised form enters through pipe 14. The reduced catalyst is separated in the area 18 of the reactor, using e.g. a nitrogen or steam flow entering through pipe 19. The dehydrogenated feed is withdrawn through pipe 16, while the reduced catalyst is collected into pipe 24 and transported to the top of the regeneration reactor 20. Gas containing molecular oxygen is introduced into reactor 20 through pipe 22 and exits through pipe 23, while the oxidized catalyst is recovered in the outlet pipe 14 and recycled into the dehydrogenation reactor 10.

The process of the present invention shows many advantages with respect to the usual processes and particularly it enables to avoid drastically the formation of oxygenated products, due to the fact that the presence of molecular oxygen is excluded. Moreover, hydrogen is eliminated upon its formation, which enables to displace the equilibrium of the reaction and to run at less high temperatures.

Therefore, it is no more necessary to use diluents such as inert gases to displace the equilibrium; however the process of the invention may also be carried out in the presence of usual diluents.

Another non-negligible advantage of the process of the invention resides in the fact that it allows to work with quasi isothermal reactors at a less high temperature, while with the prior processes it was practically necessary to work under adiabatic conditions.

A substantial increase of the conversion and the yield in dehydrogenated hydrocarbons is also noted. Further, it has been found that it was no more necessary to work under vacuum.

The process of the invention will be better illustrated by way of the following examples.

### Example 1

### A. Preparation of the catalyst

11.21 g of ammonium metavanadate were dissolved in 200 ml of hot deionised water.

30 g of magnesium oxide powder were homogeneized into 200 ml of water at 90°C during 1 hour. The metavanadate solution was then added, and the resulting mixture was homogeneized at 90°C for a further hour.

The mixture temperature was then raised to 120°C and the excess water was evaporated during 18 hours under a nitrogen flow.

The resulting solid catalyst was calcined at 600°C during 4 hours and pelletized. The pellets were ground and sieved to give a V₂O₅-type catalyst supported on magnesium oxide.

### B. Catalytic dehydrogenation of ethylbenzene

Ethylbenzene was passed in a pulse microreactor over 500 mg catalyst (as obtained from A) at a temperature of 505°C, under atmospheric pressure, the amount of ethylbenzene being of 3.6 mg per pulse.

The following results were obtained :

| | |
|---|---|
| - conversion of ethylbenzene : | 99.1 mol % |
| - styrene selectivity : | 91.7 mol % |
| - yield : | 90.9 % |

When the conversion was reduced by about 10% with regard to the initial conversion, ethylbenzene injections were stopped and the catalyst was regenerated by passing air in pulse mode at a temperature of 500°C.

After regeneration, ethylbenzene injections were resumed under the same conditions, and the following results were obtained.

| | |
|---|---|
| - conversion of ethylbenzene : | 98.1 mol % |
| - styrene selectivity : | 92.2 mol % |
| - yield : | 90.4 % |

### C. Comparative example

Using adiabatic mode, a commercial catalyst for dehydrogenation of ethylbenzene, containing iron oxide, was used at a temperature of 600°C, under a pressure of 0.06 MPa, with a liquid hourly space velocity of 0.45, and in the presence of steam in a molar ratio H₂O/ethylbenzene of 8. The following results were obtained.

| | |
|---|---|
| - conversion of ethylbenzene : | 70 mol % |
| - styrene selectivity : | 90-92 mol % |
| - yield : | 63-64.4 % |

This comparative example shows that a higher yield is obtained by the process of the invention, at lower temperatures and without having to add a diluent.

### Example 2 (comparative)

### A. Catalyst preparation

100 g of silica powder of particle size greater than 100 mesh were calcined at 500°C during 3 hours.

85 g of the calcined silica were impregnated under vacuum with a solution of iron nitrate prepared by dissolving 102.5 g of Fe(NO₃)₃.9H₂O in deionized water to obtain a final weight of 216 g.

After contact time of 18 hours at room temperature, the solid was filtered off and dried at 110°C during 18 hours. The catalyst was then placed in an oven, the temperature being increased at a rate of 30°C/h up to 600°C and maintained at 600°C during 10 hours.

The resulting catalyst was ground and sieved.

### B. Catalytic dehydrogenation of ethylbenzene

Ethylbenzene was passed in a pulse microreactor over 500 mg catalyst (as obtained from A) at a temperature of 505°C, under atmospheric pressure, the amount of ethylbenzene being of 3.6 mg per pulse.

The following results were obtained :

| | |
|---|---|
| - conversion of ethylbenzene : | 98.4 mol % |
| - styrene selectivity : | 87.2 mol % |
| - yield : | 85.8 % |

When the conversion was reduced by about 10% with regard to the initial conversion, ethylbenzene injections were stopped and the catalyst was regenerated by passing air in pulse mode at a temperature of 500°C.

After regeneration, ethylbenzene injections were resumed under the same conditions, and the following results were obtained.

| | |
|---|---|
| - conversion of ethylbenzene : | 96.5 mol % |
| - styrene selectivity : | 87.5 mol % |
| - yield : | 84.4 % |

### Example 3

### A. Preparation of the catalyst

Technical grade magnesium oxide (98 wt % MgO containing 0.8 wt % CaO, 0.3 wt % SiO₂ and 0.2 wt % Fe₂O₃ as major impurities) was used, under the form of particles (99 % of which had a size below 0.04 mm) having a specific area of 50.9 m²/g.

4.5 kg of said oxide and 3.7 kg of silicasol (containing 40.5 wt % SiO₂) were suspended in water. The suspension was spray-dried in a container kept at a temperature such that a powder was obtained having at least 70 wt % of particles between 0.04 and 0.15 mm.

The powder fraction between 0.04 and 0.15 mm was separated by sieving.

250 g of said fraction were suspended in 750 ml water. The suspension was heated to a temperature of 90°C for 1 hour. To the hot suspension, 77.2 g silicasol (40.5 wt % SiO₂) were added, then a 90°C solution of 71.8 g ammonium metavanadate in 1400 ml water.

The resulting mixture was introduced into a rotating flask kept in an oil bath and homogenised at 90°C during 15 minutes. The temperature of the oil bath was then raised to 120°C to dry the mixture maintained at that temperature and under slight nitrogen flushing for 18 hours.

The powder then obtained was sieved to retain particles between 0.04 and 0.15 mm and placed in an oven. The oven temperature was raised at a rate of 60°C/hour up to 600°C and kept at that temperature for 4 hours.

A catalyst was obtained, which was sieved to retain the particles between 0.04 and 0.15 mm. It contained 15.5 wt % of vanadium (expressed as V₂O₅), 30 % wt % of silicon (espressed as SiO₂), 1.5 wt % of sodium (expressed as Na₂O), and 0.45 wt % of calcium (expressed as CaO).

The catalyst had the following physical properties :

| | |
|---|---|
| - apparent packing density (ASTM D-4164-82) | 0.69 g/ml |
| - apparent bulk density (UOP standard method M.294-54T) | 0.58 g/ml |

| - surface area by BET technique (N₂ adsorption) | |
|---|---|
| - calcined at 500°C in air | 88 m²/g |
| - calcined at 120°C in vacuum | 53 m²/g |
| - volume of pores < 100 nm (nitrogen desorption) | 0.27 ml/g |

| - attrition loss (air jet velocity of 7.07 l/min under standard conditions) | |
|---|---|
| - from 0 to 5 hours | about 3.4 wt % |
| - from 5 to 25 hours | about 1.8 wt % |

### B. Catalytic dehydrogenation of ethylbenzene

Ethylbenzene was passed in a pulse reactor over 500 mg catalyst (as obtained from A) at a temperature of 500°C and under atmospheric pressure, the amount of ethylbenzene being of 2 mg per pulse.

The following results were obtained :

| | |
|---|---|
| - conversion of ethylbenzene | 97.6 mol % |
| - styrene selectivity | 99.4 mol % |
| - yield | 97.0 % |

Ethylbenzene injections were continued until the conversion was reduced by about 10 % with regard to its initial value. The ethylbenzene injections were then stopped and the catalyst was regenerated by passing thereon air pulses at a temperature of 500°C.

After regeneration, ethylbenzene injections were resumed under the same conditions, giving the following results :

| | |
|---|---|
| - conversion of ethylbenzene | 98.8 mol % |
| - styrene selectivity | 98.1 mol % |
| - yield | 96.9 mol % |

### Example 4

A fluidized bed reactor was used, under the following temperature conditions (described by reference to Figure 2) :

| | |
|---|---|
| - reactor | (10) 500°C |
| - regeneration | (20) 500°C |
| - stripper | (18) 500°C |
| - catalyst return | (14) 520°C |
| - feed preheater | 150°C |
| - feed entrance | (12) 370°C |

The feed and main gases flow rates were as follows (references are to Figure 2) :

| | |
|---|---|
| - ethylbenzene | (12) 1.08 mol/h |
| - nitrogen at stripper | (19) 2.31 mol/h |
| - nitrogen at regenerator | (22) 34.68 mol/h |
| - oxygen at regenerator | (22) 3.29 mol/h |

The weight hourly space velocity was of 3.4.

The catalyst used was prepared as described in part A of example 3 and calcined at 550°C immediately before use. The products (16 in Figure 2) comprised 0.57 mol/h of styrene and 0.40 mol/h of unreacted ethylbenzene, corresponding to :

| | |
|---|---|
| - ethylbenzene conversion | 63.04 mol % |
| - styrene selectivity | 83.80 mol % |
| - yield | 52.83 mol % |

### Examples 5 and 6

### A. Preparation of the catalysts

The procedure described in part A. of example 1 was repeated except that 5.99 g ammonium metavanadate were dissolved in 100 ml hot deionised water (example 5) or 1.41 g in 50 ml (example 6).

### B. Catalytic dehydrogenation of ethylbenzene

Ethylbenzene was passed in a pulse microreactor over 500 mg catalyst at a temperature of 500°C, under atmospheric pressure, the amount of ethylbenzene being of 2.1 mg per pulse, and the contact time being of about 1.3 seconds.

The following results were obtained :

| | example 5 | example 6 |
|---|---|---|
| - ethylbenzene conversion (mol %) | 96.8 | 97.4 |
| - styrene selectivity (mol %) | 94.8 | 91.9 |
| - styrene yield (mol %) | 91.8 | 89.5 |

## Claims

1. Process for the catalytic dehydrogenation of alkylaromatic hydrocarbons into the corresponding alkenylaromatic hydrocarbons, characterized in that it comprises the steps of
- contacting the hydrocarbon feedstock to be dehydrogenated under dehydrogenation conditions, in the absence of added water vapour and of any gas containing molecular oxygen, with a catalyst consisting of at least one reducible oxide of a metal (I) selected from the group consisting of V, Cr, Mn, Co, Pb, Bi, Mo, U and Sn, supported on a material selected from clays, zeolitic materials of the metallo-silicate or metallo-alumino-phosphate type, and oxides of a metal (II) selected from Ti, Zr, Zn, Th, Mg, Ca, Ba, Si and Al, said catalyst having a dehydrogenation activity when said metal (I) has a valency such that it is not in its most reduced state,
- recovering the dehydrogenated hydrocarbons,
- regenerating the used catalyst, and
- contacting the regenerated catalyst with fresh hydrocarbon feedstock to be dehydrogenated.

2. Process according to claim 1, characterized in that a catalyst comprising at least a vanadium oxide is used.

3. Process according to either of claims 1 and 2, characterized in that the catalyst is supported on magnesium oxide or on zinc oxide.

4. Process according to any one of claims 1 to 4, characterized in that the catalyst is supported on a zeolitic material selected from alumino-silicates, borosilicates and silico-alumino-phosphates.

5. Process according to any one of claims 1 to 4, characterized in that the dehydrogenation of alkylaromatic hydrocarbons and the recovery of the catalyst are carried out in a fluidized bed reactor.

6. Process according to any one of claims 1 to 4, characterized in that the supported catalyst is regenerated by passing on the catalyst bed a gaseous flow containing molecular oxygen at a temperature of from 200 to 1000°C.

7. Process according to claim 6, wherein the catalyst is regenerated when a reduction of about 10% of the conversion of alkylaromatic hydrocarbons is observed.

8. Process according to any one of claims 1 to 5, wherein the catalyst recovered from the dehydrogenation reactor is regenerated by sending it into a second reactor wherein a gaseous flow is passed at a temperature of from 200 to 1000°C, with a residence time of from 5 seconds to 5 minutes, and recovered for recycling to the dehydrogenation reactor.

9. Process according to claim 8, wherein the second reactor is of the fluidized bed type and the gaseous flow contains molecular oxygen.

10. Process according to any one of claims 1 to 9, characterized in that the dehydrogenation reaction is carried out, in the absence of gas containing molecular oxygen, at a temperature of from 360 to 800°C, preferably between 400 and 650°C, at a pressure of from 0.001 to 1 MPa, and with an hourly spatial velocity comprised between 0.01 and 20 kg of hydrocarbon per hour and per kg of catalyst.

11. Process according to any one of claims 1 to 10, wherein the catalyst is of the MgO - SiO₂ - V₂O₅ type.

12. Process according to any one of claims 1 to 11, wherein the catalyst comprises from 40 to 60 wt % of MgO, from 20 to 40 wt % of SiO₂, from 10 to 30 wt % of V₂O₅, and minor amounts of promoters and of other impurities.

13. Process according to claim 12, wherein the catalyst comprises about 50 wt % MgO, about 30 wt % SiO₂, about 15 wt % of V₂O₅, and minor amounts of promoters and of other impurities.

14. Process according to either of claims 12 and 13, wherein the catalyst is prepared by spray-drying of an MgO/SiO₂ suspension, followed by impregnation with an ammonium vanadate solution preferably also with a further amount of silica as silicasol, evaporation and calcination at a temperature of from 500°C to 800°C.

## Patentansprüche

1. Verfahren zur katalytischen Dehydrierung von alkylaromatischen Kohlenwasserstoffen zu den entsprechenden alkenylaromatischen Kohlenwasserstoffen, dadurch gekennzeichnet, daß es die Schritte umfaßt:
- Inkontaktbringen der zu dehydrierenden Kohlenwasserstoffspeisung unter Dehydrierungsbedingungen in der Abwesenheit von zugegebenem Wasserdampf und von jeglichem molekularen Sauerstoff enthaltenden Gas mit einem Katalysator, der aus mindestens einem reduzierbaren Oxid eines Metalls (I), ausgewählt aus der Gruppe, die aus V, Cr, Mn, Co, Pb, Bi, Mo, U und Sn besteht, gestützt auf einem aus Tonen, Zeolithmaterialien vom Metallsilicat- oder Metallaluminiumphosphattyp ausgewählten Material, und Oxiden eines Metalls (II), ausgewählt aus Ti, Zr, Zn, Th, Mg, Ca, Ba, Si und Al besteht, wobei der Katalysator eine Dehydrierungsaktivität aufweist, wenn das Metall (I) eine derartige Valenz aufweist, daß es sich nicht in seinem am stärksten reduzierten Zustand befindet,
- Gewinnen der dehydrierten Kohlenwasserstoffe,
- Regenerieren des gebrauchten Katalysators, und
- Inkontaktbringen des regenerierten Katalysators mit frischer, zu dehydrierender Kohlenwasserstoffspeisung.

2. Verfahren nach Anspruch 1, dadurch gekennzeichnet, daß ein mindestens ein Vanadiumoxid umfassender Katalysator verwendet wird.

3. Verfahren nach einem der Ansprüche 1 und 2, dadurch gekennzeichnet, daß der Katalysator auf Magnesiumoxid oder auf Zinkoxid gestutzt ist.

4. Verfahren nach einem der Ansprüche 1 bis 4, dadurch gekennzeichnet, daß der Katalysator auf einem aus Aluminiumsilicaten, Borsilicaten und Siliciumaluminiumphosphaten ausgewählten Zeolithmaterial gestützt ist.

5. Verfahren nach einem der Ansprüche 1 bis 4, dadurch gekennzeichnet, daß die Dehydrierung von alkylaromatischen Kohlenwasserstoffen und die Gewinnung des Katalysators in einem Fließbettreaktor durchgeführt werden.

6. Verfahren nach einem der Ansprüche 1 bis 4, dadurch gekennzeichnet, daß der gestützte Katalysator dadurch regeneriert wird, daß ein gasförmiger, molekularen Sauerstoff enthaltender Strom bei einer Temperatur von 200 bis 1000 °C über das Katalysatorbett geführt wird.

7. Verfahren nach Anspruch 6, wobei der Katalysator regeneriert wird, wenn eine Verringerung der Umwandlung von alkylaromatischen Kohlenwasserstoffen von etwa 10 % beobachtet wird.

8. Verfahren nach einem der Ansprüche 1 bis 5, wobei der aus dem Dehydrierungsreaktor gewonnene Katalysator durch Einspeisung in einen zweiten Reaktor, durch den ein gasförmiger Strom mit einer Verweilzeit von 5 Sekunden bis zu 5 Minuten bei einer Temperatur von 200 bis 1000 °C hindurchgeführt wird, regeneriert und zum Recyceln in den Dehydrierungsreaktor gewonnen wird.

9. Verfahren nach Anspruch 8, wobei der zweite Reaktor vom Typ mit einem Fließbett ist und der gasförmige Strom molekularen Sauerstoff enthält.

10. Verfahren nach einem der Ansprüche 1 bis 9, dadurch gekennzeichnet, daß die Dehydrierungsreaktion in Abwesenheit eines molekularen Sauerstoff enthaltenden Gases bei einer Temperatur von 360 bis 800 °C, vorzugsweise zwischen 400 und 650 °C bei einem Druck von 0,001 bis 1 MPa und mit einer Stundenraumgeschwindigkeit zwischen 0,01 und 20 kg Kohlenwasserstoff pro Stunde und pro kg Katalysator durchgeführt wird.

11. Verfahren nach einem der Ansprüche 1 bis 10, wobei der Katalysator vom MgO - SiO₂ - V₂O₅ Typ ist.

12. Verfahren nach einem der Ansprüche 1 bis 11, wobei der Kataysator von 40 bis 60 Gew.-% MgO, von 20 bis 40 Gew.-% SiO₂, von 10 bis 30 Gew.-% V₂O₅ und kleinere Mengen an Promotoren und anderen Verunreinigungen enthält.

13. Verfahren nach Anspruch 12, wobei der Katalysator etwa 50 Gew.-% MgO, etwa 30 Gew.-% SiO₂, etwa 15 Gew.-% V₂O₅ und kleinere Mengen an Promotoren und anderen Verunreinigungen enthält.

14. Verfahren nach einem der Ansprüche 12 und 13, wobei der Katalysator durch Sprühtrocknen einer MgO/SiO₂ Suspension, gefolgt von Imprägnieren mit einer Ammoniumvanadatlösung, vorzugsweise auch mit einer weiteren Menge an Siliciumdioxid als Silikasol, Verdampfen und Kalzinieren bei einer Temperatur von 500 °C bis 800 °C hergestellt wird.

## Revendications

1. Procédé de déshydrogénation catalytique d'hydrocarbures alkylaromatiques en hydrocarbures alkénylaromatiques correspondants caractérisé en ce qu'il comprend les étapes de :
- mettre en contact la charge d'hydrocarbures à déshydrogéner dans des conditions de déshydrogénation, en l'absence d'addition de vapeur d'eau ou de tout gaz contenant de l'oxygène moléculaire, avec un catalyseur constitué par au moins un oxyde réductible d'un métal (I) choisi parmi le groupe constitué de V, Cr, Mn, Co, Pb, Bi, Mo, U et Sn, supporté sur un matériau choisi parmi les argiles, les matériaux zéolitiques de type métallo-silicate ou métallo-alumino-phosphate, et les oxydes d'un métal (II) choisi parmi Ti, Zr, Zn, Th, Mg, Ca, Ba, Si et Al, le dit catalyseur ayant une activité de déshydrogénation lorsque le dit métal (I) a une valence telle qu'il ne soit pas en son état réduit maximum,
- récupérer les hydrocarbures déshydrogénés,
- régénérer le catalyseur usagé, et
- mettre en contact le catalyseur régénéré avec la charge fraîche d'hydrocarbures à déshydrogéner.

2. Procédé selon la revendication 1, caractérisé en ce que l'on utilise un catalyseur constitué par au moins un oxyde de vanadium.

3. Procédé selon l'une quelconque des revendications 1 et 2, caractérisé en ce que le catalyseur est déposé sur un support constitué d'un oxyde de magnésium ou d'un oxyde de zinc.

4. Procédé selon l'une quelconque des revendications 1 à 3, caractérisé en ce que le catalyseur est déposé sur un support constitué par un matériau zeolitique choisi parmi les alumino-silicates, les borosilicates et les silico-alumino-phosphates.

5. Procédé selon l'une quelconque des revendications 1 à 4, caractérisé en ce que l'on réalise la déshydrogénation des hydrocarbures alkylaromatiques et la récupération du catalyseur dans un réacteur à lit fluidisé.

6. Procédé selon l'une quelconque des revendications 1 à 4, caractérisé en ce que l'on régénère le catalyseur supporté en faisant passer sur le lit catalytique un courant gazeux contenant de l'oxygène moléculaire à une température comprise entre 200 et 1000°C.

7. Procédé selon la revendication 6 caractérisé en ce que l'on effectue la régénération du catalyseur lorsque l'on constate une réduction d'environ 10% de la conversion des hydrocarbures alkylaromatiques.

8. Procédé selon l'une quelconque des revendications 1 à 5, caractérisé en ce que l'on régénère le catalyseur récupéré du réacteur de déshydrogénation en l'envoyant dans un second réacteur dans lequel on le soumet au passage d'un courant gazeux à une température comprise entre 200 et 1000°C, avec un temps de résidence compris entre 5 secondes et 5 minutes, et en récupérant le catalyseur régénéré pour le recycler dans le réacteur de déshydrogénation.

9. Procédé selon la revendication 8, caractérisé en ce que le second réacteur est à lit fluidisé et que le courant gazeux qui le traverse contient de l'oxygène moléculaire.

10. Procédé selon l'une quelconque des revendications 1 à 9, caractérisé en ce que l'on effectue la réaction de déshydrogénation, en l'absence de gaz contenant de l'oxygène moléculaire, à une température comprise entre 360 et 800°C, de préférence entre 400 et 650°C, à une pression comprise entre 0,001 et 1 MPa et à une vitesse spatiale horaire comprise entre 0,01 et 20,0 kg d'hydrocarbure par heure et par kg de catalyseur.

11. Procédé selon l'une quelconque des revendications 1 à 10, caractérisé en ce que le catalyseur est du type MgO - SiO₂ - V₂O₅.

12. Procédé selon l'une quelconque des revendications 1 à 11, caractérisé en ce que le catalyseur comprend de 40 à 60 % en pds de MgO, de 20 à 40 % en pds de SiO₂, de 10 à 30 % en pds de V₂O₅, et des quantités mineures de promoteurs et d'autres impuretés.

13. Procédé selon la revendication 12, caractérisé en ce que le catalyseur comprend environ 50 % en pds de MgO, environ 30 % en pds de SiO₂, environ 15 % en pds de V₂O₅, et des quantités mineures de promoteurs et d'autres impuretés.

14. Procédé selon l'une quelconque des revendications 12 et 13, caractérisé en ce que le catalyseur est préparé par séchage par atomisation d'une suspension de MgO/SiO₂ , suivi d'une imprégnation avec une solution de vanadate d'ammonium, de préférence également avec une quantité supplémentaire de SiO₂ comme le silicasol, d'évaporation et de calcination à une température de 500°C à 800°C.
